## Europäisches Patentamt

## European Patent Office

## Office européen des brevets

(11) Veröffentlichungsnummer: **0 148 322**
**A1**

(12) # EUROPÄISCHE PATENTANMELDUNG

(21) Anmeldenummer: **84111061.2**

(22) Anmeldetag: **17.09.84**

(51) Int. Cl.⁴: **A 61 F 9/06**

(30) Priorität: **21.09.83 CH 5124/83**

(43) Veröffentlichungstag der Anmeldung: **17.07.85**
**Patentblatt 85/29**

(84) Benannte Vertragsstaaten: **AT BE CH DE FR GB IT LI LU NL SE**

(71) Anmelder: **BUDMIGER, Hermann, Mühlematt 114, CH-4206 Seewen (CH)**

(72) Erfinder: **BUDMIGER, Hermann, Mühlematt 114, CH-4206 Seewen (CH)**

(74) Vertreter: **Schmauder, Klaus Dieter et al, c/o Schmauder & Wann Patentanwaltsbüro Nidelbadstrasse 75, CH-8038 Zürich (CH)**

(54) **Augenschutzvorrichtung.**

(57) Das Augenschutzgerät enthält ein in einem Gehäuse (2) angeordnetes Sichtfenster (10), in dem eine Sichtscheibe (12) und ein Schweißerschutzfilter (18) angeordnet sind. Das Schweißerschutzfilter enthält einen ersten, feststehenden Scheibenteil (20) und einen zweiten Scheibenteil (22), der mittels eines Antriebes (26) aus einer Sperrstellung in eine Freistellung und umgekehrt bewegbar ist. Zur Steuerung dient eine Steuervorrichtung (36), die primär mittels eines manuellen Schaltgliedes (46) aktiviert wird und als Noteinrichtung einen elektro-optischen Sensor (44) aufweist, der auf Schweißlicht anspricht.

EP 0 148 322 A1

Augenschutzgerät für eine Schweisserschutzeinrichtung
_____

Die Erfindung betrifft ein Augenschutzgerät für eine Schweisserschutzeinrichtung, insbesondere für ein Schweisserschutzschild oder eine -haube gemäss Oberbegriff des Anspruches
1.

Augenschutzgeräte der eingangs genannten Art sind bekannt,
so beispielsweise durch die Schweisserschutz-Maske und -Schil-
de der Revue Thommen AG. Das Sichtfenster dient zur direkten
Beobachtung einer Schweissstelle und enthält eine Sichtscheibe, welche in der Regel einen ersten Scheibenteil aufweist,
der UV-Strahlen zurückhält und einen zweiten Scheibenteil,
der IR-Strahlen zurückhält. Weiter ist in dem Sichtfenster
ein Schweisserschutzfilter angeordnet, welches aus Flüssigkristallen gebildet ist, die mit einer Steuervorrichtung verbunden sind, welche von einem elektrooptischen Sensor gesteuert wird. Beim Auftreten von Schweisslicht legt die Steuervorrichtung eine Spannung an das Schweisserschutzfilter, worauf die Flüssigkristalle ihre optischen Eingenschaften ändern und für das Schweisslicht undurchlässig werden. Mit diesem Augenschutzgerät werden sehr gute Ergebnisse erzielt,
jedoch enthalten sie einerseits den Nachteil, dass das entsprechende Sichtfenster aus Kostengründen nur relativ klein
gehalten werden kann, sodass es in der Regel nur einen Teilbereich des Sichtsfensters ausfüllen kann, während ein anderer Teilbereich mit einem üblichen Schweisserschutzglas versehen ist. Weiter ändern die Flüssigkristalle ihre optischen
Eigenschaften in Abhängigkeit von der Temperatur, wobei sie
insbesondere bei tieferen Temperaturen verlangsamte Schaltzeiten aufweisen, sodass die gewünschte Sicherheit nicht mehr
gegeben ist. Im übrigen ist die Durchsichtigkeit solcher Flüssigkristalle bei normalem Licht relativ schlecht.

Aufgabe der Erfindung ist es, ein Augenschutzgerät der eingangs genannten Art so auszubilden, dass in jedem Falle das Schweisserschutzfilter so schnell wirksam gemacht werden kann, dass kein Schweisslicht in die Augen des Benützers gelangen kann.

Die gestellte Aufgabe wird durch die kennzeichnenden Merkmale des Anspruches 1 gelöst.

Da ein Schweisser in der Regel vorausweiss, wann der Schweissvorgang einsetzt, kann er mit Hilfe des manuellen Schaltglides die Steuervorrichtung unmittelbar vor Beginn des Schweissvorganges aktivieren, sodass das Schweisserschutzfilter rechtzeitig im Einsatz steht, wenn der Schweissvorgang beginnt. Der elektro-optische Sensor dient in Verbindung mit der Steuervorrichtung lediglich als Notfalleinrichtung, wenn der Schweisser sich über den Beginnn des Schweissvorganges verschätzt haben sollte. Damit wird ein besonders wirksames Augenschutzgerät geschaffen, welches einen optimalen Schutz für den Benützer bietet.

Der Begriff manuelles Schaltglied soll in weitester Form verstanden werden und jede durch einen Benützer mögliche Beeinflussung beinhalten.

Vorteilhafte Ausgestaltungen des Augenschutzgerätes sind in den Ansprüchen 2 bis 13 definiert.

Das manuelle Schaltglied kann beispielsweise ein Fussschalter sein, der vom Fuss eines Bemützers betätigt wird. Auch ein Handschalter ist denkbar, der beispielsweise am Handgriff eines Elektrodenhalters der Schweissvorrichtung angeordnet sein kann. Vorteilhafter ist es, wenn das manuelle Schaltglied von einer Hand- und/oder Fussbedienung frei ist.

Dementsprechend kann das manuelle Schaltglied beispielsweise nach Anspruch 2 ausgebildet sein, wobei der akustische Sensor zweckmässigerweise in einem Schweisserschutzschild oder einer Schweisserschutzhaube in dem Bereich angeordnet ist, welcher dem Mund des Benützers gegenüber steht. Ein solcher akustischer Sensor kann dann auf bestimmte Laute ansprechen. Vorteilhafter ist noch eine Ausbildung nach Anspruch 3, da dann der Benützer lediglich in den pneumatischen Sensor zu blasen hat. Eine besonders zweckmässige Ausgestaltung des pneumatischen Sensors umschreibt Anspruch 4.

Während das Aktivieren des Schweisserschutzfilters primär über das manuelle Schaltglied erfolgt, geschieht das Passivieren zweckmässigerweise nach Anspruch 5. Durch das Zeitglied wird sichergestellt, dass kurzzeitige Unterbrechungen des Schweissvorganges nicht erfasst werden.

Das Augenschutzgerät kann nach Anspruch 6 ausgestaltet sein. Vorteilhafter ist jedoch eine Ausbildung nach Anspruch 7, da dadurch die Unzulänglichkeiten eines Flüssigkristallfilters ausgeschaltet werden. Ein als Schutzglas ausgebildetes Schweisserschutzfilter ermöglicht die Anordnung des Schutzglases über den ganzen Bereich eines Sichtfensters und behält seine Schutzeigenschaften unabhängig von den Umgebungsbedingungen bei. Ein solches Schweisserschutzfilter kann aus einer dünnen Kunststoff- oder Glasplatte bestehen, die entsprechend dem gewünschten Transmissionsgrad eingefärbt und verspiegelt ist; ferner kann sie aus zwei Scheibenteilen bestehen, deren Polarisationsebenen um $90^{\circ}$ zueinanderstehen. Die Betätigung eines solchen Schutzglases kann nach Anspruch 8 erfolgen.

Vorteilhafter ist jedoch eine Ausbildung des Augenschutzgerätes nach Anspruch 9, wodurch sich die Schaltzeiten für das
Schutzglas weiter verkürzen lassen. Die Ansprüche 10 und 11
beschreiben sehr einfache und zweckmässige Ausgestaltungen
des Antriebes. Die Ansprüche 12 und 13 definieren sehr vorteilhafte Ausführungsvarianten der Vorspannung des Schutzglases. Die Steuerung des Verschiebeweges eines solchen Schutzglases kann auf elektrischem Wege geschehen, indem beispielsweise ein Zeitglied die Einschaltzeit des Antriebes begrenzt.

Vorteilhafte Ausführungsbeispiele des erfindungsgemässen Augenschutzgerätes werden nachfolgend anhand schematischer
Zeichnungen näher beschrieben, dabei zeigen:

Figur 1        ein Augenschutzgerät in Ansicht auf die
               Rückseite, mit teilweise abgenommenem
               Deckel;

Figur 2        das Augenschutzgerät der Figur 1 im
               Schnitt II-II der Figur 1;

Figur 3        ein weiteres Augenschutzgerät im Blockauf-
               bau mit einem Flüssigkristallfilter, im
               Vertikalschnitt;

Figur 4        den Antrieb eines Augenschutzgerätes im
               Vertikalschnitt;

Figur 5        einen weiteren Antrieb eines Augenschutz-
               gerätes im Vertikalschnitt; und

Figur 6        den Antrieb und die Vorspannvorrichtung
               eines weiteren Augenschutzgerätes, in Ansicht auf die Rückseite und teilweise geschnitten.

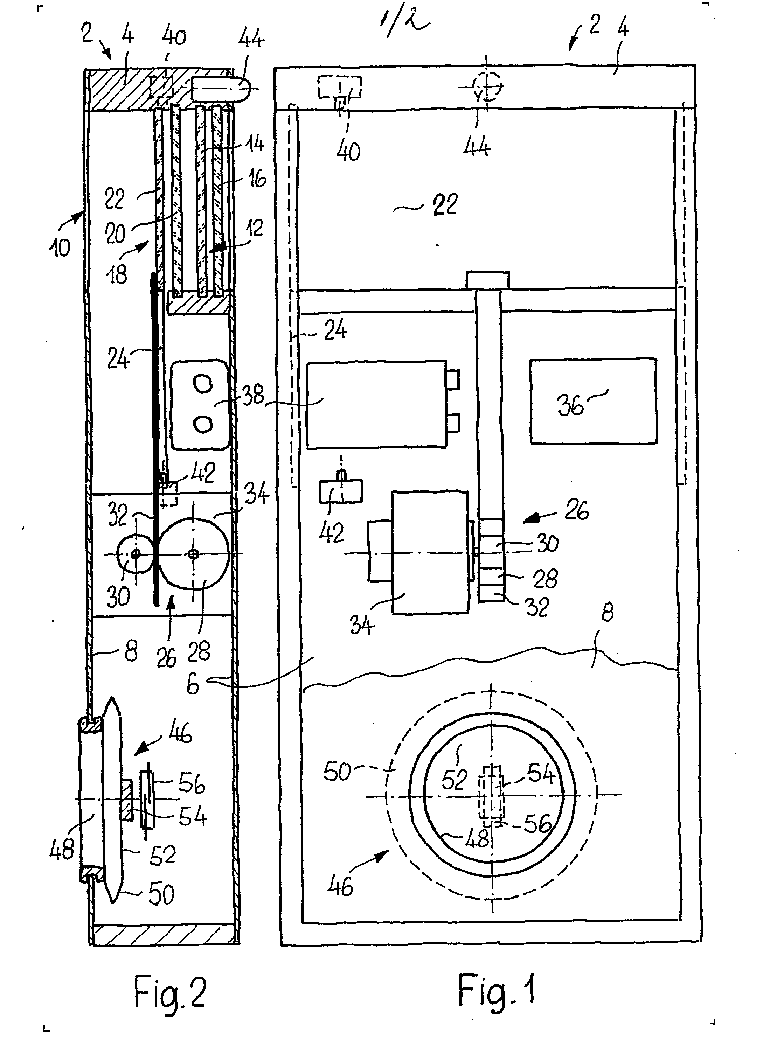

Die Figuren 1 und 2 zeigen ein Augenschutzgerät für eine
Schweisserschutzeinrichtung, insbesondere für ein Schweisserschutzschild oder eine Schweisserschutzhaube. Das Augenschutzgerät enthält ein Gehäuse 2 mit einem Rahmen 4, einer Grundplatte 6 und einer Deckplatte 8. Im Gehäuse ist ein Sichtfenster 10 angeordnet, in dem eine Sichtscheibe 12 eingebaut
ist. Diese besteht aus einem ersten Glasscheibenteil 14 zum
Abhalten des UV-Strahlenanteiles, sowie aus einem zweiten
Glasscheibenteil 16 zum Abhalten des IR-Strahlenanteiles.
Weiter ist in dem Sichtfenster 10 ein Schweisserschutzfilter
18 angeordnet, welches aus einem ersten Scheibenteil 20 gebildet ist, der feststehend ist, sowie aus einem zweiten
Scheibenteil 22, der in einer vertikalen Führung 24 auf und
ab verschiebbar ist. Die Scheibenteile 20, 22 sind Polarisationsfilter, deren Polarisationsebenen senkrecht aufeinander
stehen.

Zum Verschieben des zweiten Scheibenteiles 22 des Schweisserschutzfilters 18 dient ein Antrieb 26 mit einem Reibrad 28
und einer Druckrolle 30, zwischen denen eine Reibstange 32
eingeführt ist, die mit dem Scheibenteil 22 verbunden ist.
Der Antrieb 26 enthält einen polumschaltbaren Antriebsmotor
34, der mit einer Steuervorrichtung 36 verbunden ist, die
von einer Batterie 38 gespeist wird. An der Steuervorrichtung
36 sind Endschalter 40, 42 zur Begrenzung des Verschiebeweges
des Scheibenteiles 22 angeschlossen, sowie eine elektro-optischer Sensor 44, der nur auf Schweisslicht anspricht und
die Steuervorrichtung 36 aktiviert. Durch geeignete, nicht
näher dargestellte Filter wird der Einfluss von Fremdlicht
eliminiert.

- 8 -

6

An der Steuervorrichtung 36 ist ferner ein manuelles Schaltglied 46 angeschlossen, welches als pneumatischer Schalter ausgestaltet ist und auf Blasluft eines Benützers anspricht. Das Schaltglied 46 enthält einen an einer Oeffnung 48 der Deckplatte 8 angeschlossenen Balg 50, an dem eine Membrane 52 angeordnet ist. Letztere trägt einen Permanentmagneten 54, der bei Annäherung einen Reedschalter 56 betätigt, welcher mit der Steuervorrichtung 36 verbunden ist.

Die Funktionsweise des Augenschutzgerätes ist wie folgt:

Der zweite Scheibenteil 22 des Schweisserschutzfilters 18 steht im Normalfall in der in Figur 2 dargestellten Freistellung ausserhalb des Sichtfensters 10. Durch das Sichtfenster 10 kann ein Benützer den zu schweissenden Gegenstand beobachten und seine Schweisselektrode in die zum Schweissen geeignete Lage bringen. Unmittelbar vor Beginn des Schweissvorganges bläst der Benützer in das manuelle Schaltglied 46, worauf der Permanentmagnet 54 dem Reedschalter 56 genähert wird und diesen schliesst. Darauf hin wird die Steuervorrichtung 36 aktiviert und der Antrieb 26 eingeschaltet. Das Reibrad 28 bewegt die Reibstange 32 und damit den zweiten Scheibenteil 22 längs der Führung 24 in die Sperrstellung im Sichtfenster. Sobald der Scheibenteil 22 den Endschalter 40 erreicht hat, stellt der Antrieb 26 ab. Solange der Sensor 44 Schweisslicht feststellt, bleibt die Steuervorrichtung aktiv. Bei einem längeren Unterbruch des Schweisslichtes wird über ein nicht näher dargestelltes Zeitglied in der Steuervorrichtung 36 ein Steuersignal erzeugt, welches den Antriebsmotor 34 umschaltet, sodass der zweite Scheibenteil 22 nach unten bewegt wird, bis er am unteren Endschalter 42 ansteht.

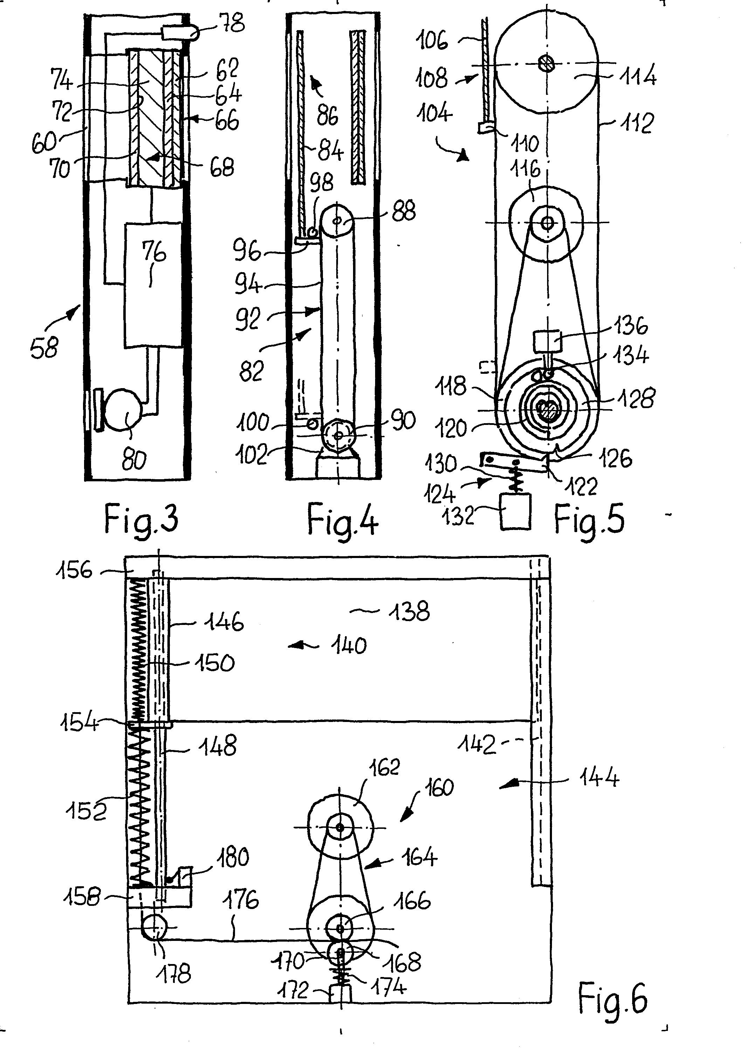

In Figur 3 ist ein weiteres Augenschutzgerät in schematischer Darstellung gezeigt, welches in einem Gehäuse 58 ein Sichtfenster 60 enthält, in dem eine aus einem ersten Glasscheibenteil 62 zur Abschirmung der IR-Strahlen, und einem zweiten Glasscheibenteil 64 zur Abschirmung von UV-Strahlen gebildete Sichtscheibe 66 angeordnet ist. In Strahlendurchgangsrichtung gesehen schliesst sich ein Schweisserschutzfilter 68 an, welches eine Stützscheibe 70 enthält, die mit der Sichtscheibe 66 eine Kammer 72 bildet, in der Flüssigkristalle 74 angeordnet sind, die in Abhängigkeit von der angelegten Spannung einer Steuervorrichtung 76 ihre optischen Eigenschaften ändern. Das Augenschutzgerät enthält weiter einen elektro-optischen Sensor 78, der auf Schweisslicht anspricht und mit der Steuervorrichtung 76 verbunden ist. Ferner ist ein manuelles Schaltglied 80 vorhanden, das als Mikrophon ausgebildet ist und ebenfalls mit der Steuervorrichtung 76 verbunden ist.

Auch bei diesem Augenschutzgerät wird das Schweisserschutzfilter 68 primär durch einen Laut aktiviert, der vom manuellen Schaltglied 80 empfangen wird. Der elektro-optische Sensor 78 hat bezüglich der Aktivierung des Schweisserschutzfilters 68 nur eine Hilfsfunktion und dient überdies zum Passivieren des Schweisserschutzfilters 68, wenn das Schweisslicht eine bestimmte Zeit unterbrochen ist.

Die Figur 4 zeigt einen abgewandelten Antrieb 82 für ein als Schutzscheibe 84 ausgebildetes Schweisserschutzfilter 86. Die Schutzscheibe 84 kann aus Kunststoff oder Glas bestehen, eingefärbt und versilbert sein. Der Antrieb 82 enthält ein zwischen einer Umlenkrolle 88 und einer Antriebsrolle 90 geführtes Umlauforgan. An einem Trum 94 des Umlauforganes 92 ist

ein Mitnehmer 96 angeordnet, der mit der Schutzscheibe 84 verbunden ist. Der Mitnehmer 96 ist zwischen zwei Endanschlägen 98, 100 hin und her verfahrbar. Ein polumschaltbarer Antriebsmotor 102 ist mit einer nicht näher dargestellten Steuervorrichtung verbunden. Der durch das Anlaufen an den Endanschlägen 98, 100 entstehende Ueberstrom wird zum Ausschalten des Antriebsmotors 102 verwendet. Im übrigen kann das Augenschutzgerät entsprechend den vorgehend beschriebenen Augenschutzgeräten ausgerüstet sein und funktionieren.

Die Figur 5 zeigt einen weiter abgewandelten Antrieb 104 eines Augenschutzgerätes. Auch hier ist die verschiebbare Schutzscheibe 106 eines Schweisserschutzfilters 108 mit dem Mitnehmer 110 eines Umlauforganes 112 verbunden. Letzteres ist wiederum über eine Umlenkrolle 114 und eine von einem Motor 116 angetriebene Antriebsrolle 118 geführt.

Dieser Antrieb 104 ist nur in Oeffnungsrichtung der Schutzscheibe 106 wirksam, wobei eine mit der Antriebsrolle 118 verbundene Spiralfeder 120 gespannt wird. In der gestrichelt angedeuteten Freistellung rastet eine Klinke 122 einer Rastenvorrichtung 124 in eine Ausnehmung 126 einer Steuerscheibe 128 ein, die mit der Antriebsrolle 118 verbunden ist. Die Klinke 122 ist mittels einer Feder 130 gegen die Steuerscheibe 128 vorgespannt. Ein von einer nicht näher dargestellten Steuervorrichtung betätigbarer Elektromagnet 132 zieht die Klinke 122 an, wenn über ein ebenfalls nicht näher dargestelltes Schaltglied oder einen elektro-optischen Sensor ein Impuls an die Steuervorrichtung abgegeben wird, wie dies im Zusammenhang mit den anderen Augenschutzgeräten beschrieben ist. Dadurch wird die gespannte Spiralfeder 120 freigegeben

und bewegt die verschiebbare Schutzscheibe 106 des Schweisserschutzfilters 108 in die Sperrstellung. Die Oeffnungsbewegung
mit Hilfe des Motors 116 wird durch einen an der Steuerscheibe 128 angeordneten Schaltnocken 134 begrenzt, der auf einen
Endschalter 136 wirkt, wenn die in Figur 5 gestrichelt gezeigte Freistellung erreicht ist. Der Verschiebeweg beim Oeffnen
des Scheibenteiles 106 entspricht praktische einer Umdrehung
der Antriebsrolle 118 und damit der Steuerscheibe 128.

Die Figur 6 zeigt ein weiteres Ausführungsbeispiel eines Augenschutzgerätes bei dem die verschiebbare Schutzscheibe 138
eines Schweisserschutzfilters 140 auf einer Seite in einer
Führungsnut 142 des Gehäuses 144 geführt ist. Die andere Seite der Schutzscheibe 138 ist mit einer Führungsbuchse 146
versehen, die auf einer vertikalen Führungsachse 148 verschiebbar ist. Die Schutzscheibe 138 ist mittels einer Zugfeder 150 und einer Druckfeder 152 in Sperrstellung vorgespannt. Die Zugfeder 150 ist zwischen einem Mitnehmer 154,
der am unteren Ende der Führungsbuchse 146 angeordnet ist
und einer oberen Halterung 156 des Gehäuses 144 eingespannt.
Die Druckfeder 152 stützt sich an ihrem oberen Ende auf der
Unterseite des Mitnehmers 154 und einer unteren Halterung
158 des Gehäuses 144 ab.

Der Antrieb 160 des Augenschutzgerätes enthält einen Motor
162, der über einen Riementrieb 164 ein Reibrad 166 antreibt.
Eine Druckrolle 168 wird vom Anker 170 eines Elektromagneten
172 gehalten und mittels einer Druckfeder 174 gegen das Reibrad 166 vorgespannt. Zwischen dem Reibrad 166 und der Druckrolle 168 ist eine Reibband 176 eingespannt, das über eine
Umlenkrolle 178 mit dem Mitnehmer 154 der Schutzscheibe 138
verbunden ist.

Zum Oeffnen der Schutzscheibe 138 zieht der Antrieb 160 über
das Reibrad 166 das Reibband 176, wodurch die Schutzscheibe
138 unter Spannung der Zugfeder 150 und der Druckfeder 152
nach unten bewegt wird, bis die Führungsbuchse 146 einen Endschalter 180 betätigt, der den Motor 162 abstellt. Der ausgeschaltete Antrieb 160 hält die Schutzscheibe 138 in der
Freistellung. Eine nicht näher dargestellte Steuereinrichtung
mit elektro-optischem Sensor und manuellem Schaltglied, die
entsprechend den obigen Ausführungsbeispielen ausgestaltet
sind, steuern das Augenschutzgerät. Bei Betätigung des manuellen Schaltgliedes oder elektro-optischen Sensors wird die
Druckrolle 168 mittels des Elektromagneten 172 von dem Reibrad 166 abgehoben, wodurch das Reibband 176 freikommt und die
Schutzscheibe 138 aufgrund der Vorspannung durch die Zugfeder
150 und die Druckfeder 152 unmittelbar in Sperrstellung bewegt wird.

Es sind noch die verschiedensten Ausführungsformen des Augenschutzgerätes möglich. So kann beispielsweise bei dem Augenschutzgerät der Figur 6 nur eine Druckfeder oder eine Zugfeder zur Anwendung kommen. Auch kann die andere Seite der verschiebbaren Schutzscheibe mit einer Führungsbuchse, Führungsachse und Vorspannfeder versehen sein. Ferner sind einzelne
Bestandteile der oben beschriebenen Augenschutzgeräte untereinander austauschbar.

Bezugszeichenliste

| | |
|---|---|
| 2 | Gehäuse |
| 4 | Rahmen |
| 6 | Grundplatte |
| 8 | Deckplatte |
| 10 | Sichtfenster |
| 12 | Sichtscheibe |
| 14 | 1. Glasscheibenteil für UV |
| 16 | 2. Glasscheibenteil für IR |
| 18 | Schweisserschutzfilter |
| 20 | 1. Scheibenteil |
| 22 | 2. Scheibenteil |
| 24 | vertikale Führung |
| 26 | Antrieb |
| 28 | Reibrad |
| 30 | Druckrolle |
| 32 | Reibstange |
| 34 | Antriebsmotor |
| 36 | Steuervorrichtung |
| 38 | Batterie |
| 40 | Endschalter |
| 42 | Endschalter |
| 44 | Sensor |
| 46 | manuelles Schaltglied |
| 48 | Oeffnung |
| 50 | Balg |
| 52 | Membrane |
| 54 | Permanentmagnet |
| 56 | Reedschalter |

| 58 | Gehäuse |
|-----|---------|
| 60 | Sichtfenster |
| 62 | 1. Scheibenteil |
| 64 | 2. Scheibenteil |
| 66 | Sichtscheibe |
| 68 | Schweisserschutzfilter |
| 70 | Stützscheibe |
| 72 | Kammer |
| 74 | Flüssigkristall |
| 76 | Steuervorrichtung |
| 78 | Sensor |
| 80 | manuelles Schaltglied |
| 82 | Antrieb |
| 84 | Schutzscheibe |
| 86 | Schweisserschutzfilter |
| 88 | Umlenkrolle |
| 90 | Antriebsrolle |
| 92 | Umlauforgan |
| 94 | Trum |
| 96 | Mitnehmer |
| 98 | Endanschlag |
| 100 | Endanschlag |
| 102 | Antriebsmotor |
| 104 | Antrieb |
| 106 | Schutzscheibe |
| 108 | Schweisserschutzfilter |
| 110 | Mitnehmer |
| 112 | Umlauforgan |
| 114 | Umlenkrolle |
| 116 | Motor |
| 118 | Antriebsrolle |
| 120 | Spiralfeder |

0148322

- 16 -

| 122 | Klinke |
| 124 | Rastenvorrichtung |
| 126 | Ausnehmung |
| 128 | Steuerscheibe |
| 130 | Feder |
| 132 | Elektromagnet |
| 134 | Schaltnocken |
| 136 | Endschalter |
| 138 | Schutzscheibe |
| 140 | Schweisserschutzfilter |
| 142 | Führungsnut |
| 144 | Gehäuse |
| 146 | Führungsbuchse |
| 148 | Führungsachse |
| 150 | Zugfeder |
| 152 | Druckfeder |
| 154 | Mitnehmer |
| 156 | Halterung (oben) |
| 158 | Halterung (unten) |
| 160 | Antrieb |
| 162 | Motor |
| 164 | Riementrieb |
| 166 | Reibrad |
| 168 | Druckrolle |
| 170 | Anker |
| 172 | Elektromagnet |
| 174 | Druckfeder |
| 176 | Reibband |
| 178 | Umlenkrolle |
| 180 | Endschalter |

Patentansprüche

1. Augenschutzgerät für eine Schweisserschutzeinrichtung, insbesondere für ein Schweisserschutzschild oder eine -haube, mit einem ein Sichtfenster enthaltenden Gehäuse, wobei im Sichtfenster eine Sichtscheibe und ein Schweisserschutzfilter angeordnet sind, welches mittels einer, einen elektro-optischen Sensor enthaltenden Steuervorrichtung schaltbar ist, dadurch gekennzeichnet, dass die Steuervorrichtung (36, 76) zusätzlich ein manuelles Schaltglied (46, 80) zum Aktivieren des Schweisserschutzfilters (18, 68, 86, 108, 140) aufweist.

2. Augenschutzgerät nach Anspruch 1, dadurch gekennzeichnet, dass das manuelle Schaltglied (80) als akustischer Sensor ausgebildet ist, der vorzugsweise auf bestimmte akustische Zeichen anspricht.

3. Augenschutzgerät nach Anspruch 1, dadurch gekennzeichnet, dass das manuelle Schaltglied (46) als pneumatischer Sensor ausgebildet ist, der auf einen geblasenen Luftstrom anspricht.

4. Augenschutzgerät nach Anspruch 3, dadurch gekennzeichnet, dass der pneumatische Sensor (46) eine Membrane (52) aufweist, die mit einem Schalter (56), vorzugsweise einem Reedschalter, zusammenwirkt, wobei die Membrane (52) vorzugsweise einen Permanentmagneten (54) trägt.

P-1331
Augenschutzvorrichtung
29.08.1983

5. Augenschutzgerät nach Anspruch 1, dadurch gekennzeichnet, dass die Steuereinrichtung (36, 76) ein Zeitglied aufweist, welches beim Ausbleiben des Schweisslichtes das Schweisserschutzfilter nach einer bestimmten Zeitspanne passiviert.

6. Augenschutzgerät nach Anspruch 1, dadurch gekennzeichnet, dass das Schweisserschutzfilter (68) ein Flüssigkristallfilter ist.

7. Augenschutzgerät nach Anspruch 1, dadurch gekennzeichnet, dass das Schweisserschutzfilter (18, 86, 108, 140) eine vor die Sichtscheibe (12) verfahrbare Schutzscheibe (22, 84, 106, 138) aufweist.

8. Augenschutzgerät nach Anspruch 7, dadurch gekennzeichnet, dass die Schutzscheibe (22, 84) mittels eines Antriebes (26, 82) sowohl in die Sperrstellung wie in Freistellung verfahrbar ist.

9. Augenschutzgerät nach Anspruch 7, dadurch gekennzeichnet, dass die Schutzscheibe (106, 138) mittels eines Antriebes (104, 160) in Freistellung verfahrbar ist und mittels einer lösbaren Arretierung (122, 124; 168, 174) in der Freistellung gehalten und mittels mindestens einer Feder (120, 150, 152) in die Sperrstellung vorgespannt ist.

10. Augenschutzgerät nach Anspruch 7, dadurch gekennzeichnet, dass der Antrieb (26, 160) ein mittels eines Reibrades (28, 166) antreibbares Reibglied (32, 176), beispielsweise eine Reibstange (32) oder ein Reibband (176) aufweist.

11. Augenschutzgerät nach Anspruch 7, dadurch gekennzeichnet, dass der Antrieb (82, 104) ein angetriebenes Umlauforgan (92, 112) aufweist, an dessen einem Trum (94) ein Mitnehmer (96, 110) für die Schutzscheibe (84, 104) angeordnet ist.

12. Augenschutzgerät nach Anspruch 9, dadurch gekennzeichnet, dass die Vorspannfeder eine mit einer Antriebsrolle (118) verbundene Spiralfeder (120) ist.

13. Augenschutzgerät nach Anspruch 9, dadurch gekennzeichnet, dass zur Vorspannung parallel zum Verschiebeweg der Schutzscheibe (138) eine Zugfeder (150) und/oder Druckfeder (152) angeordnet sind/ist.

Fig.2          Fig.1

0148322

Fig.3

Fig.4

Fig.5

Fig.6

EP 84111061.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US - A - 4 241 286 (GORDON)<br>* Patentansprüche 1,2 *<br>-- | 1,2,6 | A 61 F 9/06 |
| A | DE - A1 - 2 553 976 (BUDMIGER)<br>* Ansprüche 1,2,8 *<br>-- | 1,6 | |
| A | DE - C - 725 672 (GOODE)<br>* Patentansprüche 1,2 *<br>-- | 1;3 | |
| A | CH - A - 407 364 (BURMEISTER)<br>* Patentanspruch, Unteranspruch 6 *<br>---- | 1,7,9 | |

RECHERCHIERTE SACHGEBIETE (Int. Cl.4)

A 61 F
B 23 K
H 02 P

Der vorliegende Recherchenbericht wurde für alle Patentansprüche erstellt.

| Recherchenort | Abschlußdatum der Recherche | Prüfer |
|---|---|---|
| WIEN | 12-03-1985 | MIHATSEK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82